# EUROPEAN PATENT APPLICATION

(11) **EP 2 022 399 A1**
(43) Date of publication of application: **11.02.2009**
(21) Application number: 07744414.9
(22) Date of filing: 30.05.2007
(51) Int. Cl.: A61B 5/15, A61B 5/151

(54) **PUNCTURE NEEDLE AND LANCET HAVING IT**

(30) Priority: 02.06.2006 JP 2006154487
(71) Applicant: Asahi Polyslider Company, Limited, Osaka 530-0005 (JP)
(72) Inventor: TAMESADA, Yuji, Maniwa-shi, Okayama 719-3226 (JP)
(74) Representative: HOFFMANN EITLE
(86) International application number: PCT/JP2007/061001
(87) International publication number: WO 2007/142098

(57) **Abstract**

There is provided a pricking needle which causes less pain upon the pricking operation.
The front end portion of the pricking needle is defined by:
(1) a rear portion of an ellipse which is formed by cutting through the cylindrical member obliquely ahead from its backward;
(2) an isosceles triangle portion that has a central portion of a front edge of the rear portion of the ellipse as a base, that extends forward and obliquely downward from the central portion, and that extends more obliquely downward than a spreading direction of the rear portion of the ellipse;
(3) a rightward inclined surface portion and a leftward inclined surface portion, each including an oblique side of the isosceles triangle portion, a side portion of the front edge of the rear portion of the ellipse and a line segment which connects a front apex of the isosceles triangle portion and a distal end of the pricking needle, and extending from the right oblique side of the isosceles triangle obliquely downward; and
(4) a portion of a side face of the cylindrical member.

## Description

### TECHNICAL FIELD

The present invention relates to a needle used in a lancet for obtaining an amount of a body fluid, particularly blood, and particularly to a pricking (or pricking) needle and a lancet that has the same

### BACKGROUND ART

In order to measure a glucose concentration in blood, namely a blood sugar level, it may be required to obtain a blood sample. To obtain an amount of a blood sample, a sharp member such as a needle is used to prick a predetermined portion of a body such as a finger tip, an ear lobe or a heel so that the portion bleeds. Various pricking devices have been used in the operations to obtain the blood sample as described above.

Such devices are generally called lancets, and employ metal needles of various sizes in thickness as the pricking member. When subjected to the pricking operation, a person feels a pain. The pain felt upon being pricked may be increased by the psychological tension due to anticipation of being pricked, and it is therefore required to reduce the pain as much as possible.

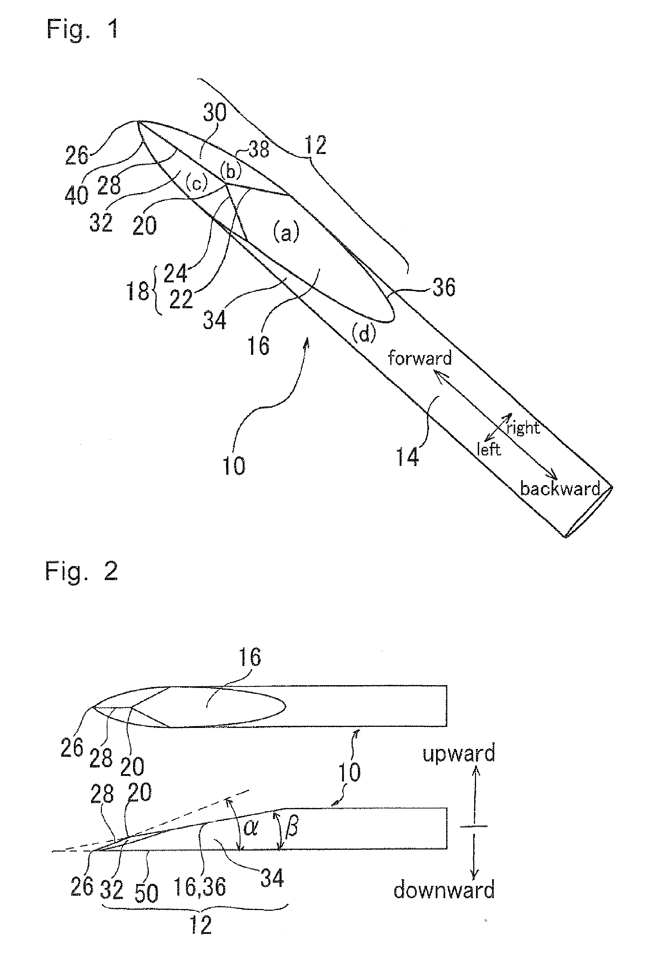

Accordingly, a pricking needle 10 that has such a front end portion as shown in Fig. 1 is used today, in consideration of both the pricking operation itself and the mitigation of pain. At least a part of the front end portion 12 of the pricking needle 10 constitutes a pricking region (that is, a portion of a pricking member which portion is located in the front of the pricking member) that intrudes at a predetermined point into the body. The pricking region may be either the entire front end portion 12 or a part of the front end portion 12, and in a further embodiment, the pricking region may include a portion behind the front end portion.

The front end portion 12 is composed of four surfaces; that is, three flat surfaces and one curved surface, as described below:
(a) a rear portion 16 of an ellipse which is formed by cutting through a cylindrical member 14 obliquely ahead from its backward, with a front edge 18 of the rear portion 16 consisting of a right oblique side 22 and a left oblique side 24 that define an apex 20, that is, the flat surface (a);
(b) a rightward inclined surface portion 30 which is defined by a line segment 28 as an oblique side connecting the apex 20 of the front edge 18 of the rear portion 16 of the ellipse and a distal end 26 of the pricking needle, and the right oblique side 22 of the front edge 18 of the rear portion 16 of the ellipse, and which extends from the line segment 28 obliquely downward toward the right, that is, the flat surface (b);
(c) a leftward inclined surface portion 32 which is defined by the line segment 28 as the oblique side connecting the apex 20 of the front edge 18 of the rear portion 16 of the ellipse and the distal end 26 of the pricking needle, and the left oblique side 24 of the front edge 18 of the rear portion 16 of the ellipse, and which extends from the line segment 28 obliquely downward toward the left, that is, the flat surface (c); and
(d) a portion 34 of a side face of the cylindrical member, namely the curved surface (d), which includes as its peripheral portions,
   (d-1) a peripheral portion 36 of the rear portion 16 of the elliptical rear portion 16 with excluding the front edge 18;
   (d-2) an outer peripheral portion 38 of the rightward inclined surface portion 30 (i.e. a peripheral portion located at the outermost of the rightward inclined surface portion 30 in Fig. 1) with excluding the right oblique side 22 of the rightward inclined surface portion 30 and the line segment 28; and
   (d-3) an outer peripheral portion 40 of the leftward inclined surface portion 32 (i.e. a peripheral portion located at the outermost of the leftward inclined surface portion 32 in Fig. 1) with excluding the left oblique side 24 of the leftward inclined surface portion 32 and the line segment 28.

It is noted a pricking needle that causes less pain during the pricking operation and has a polygonal transverse cross section is disclosed in below Patent Reference 1. While the above described pricking needle currently used has a cross section that includes a partially circular portion, the pricking needle disclosed in Patent Reference 1 has a polygonal cross section such as a square, a triangle or a rectangular.
[Patent Reference 1] Japanese Patent Unexamined Publication (Kokai) No. 2004-24878

### DISCLOSURE OF THE INVENTION

### PROBLEM TO BE SOLVED BY THE INVENTION

Even when the lancet that employs such a pricking needle as shown in Fig. 1 is used, a pain is inevitably felt during the pricking operation, and it is desired to further alleviate the pain felt during the pricking operation. Although Patent Reference 1 aims to alleviate the pain felt during the pricking operation, there is no description at all as to the mechanism whereby the disclosed pricking needle can alleviate the pain.

### MEANS TO SOLVE THE PROBLEM

In view of the background art and the problem to be solved as described above, the inventor of the present invention conducted studies as to the pricking needle. As a result, the present invention has been completed on the basis of a finding that when the rear portion (a), which consists of one flat surface, of the ellipse in the pricking needle shown in Fig. 1 is formed of bent two flat surfaces, the length of a portion that splays with a predetermined pricking angle from the distal end 26 of the pricking needle can be decreased, so that the pain felt during the pricking operation can be mitigated.

Fig. 2 is a schematic side view of the pricking needle shown in Fig. 1. In Fig. 2, the pricking needle 10 has a front end portion 12 as the pricking region, in which the distal end 26 of the pricking region is a tip of the pricking needle, which constitutes a sharp apex that starts the pricking into a predetermined portion. The pricking angle of the pricking needle 10 corresponds to the angle α formed by a base portion 50 of the pricking needle (the distal end 26 of the pricking needle corresponds to one end of the base portion) and an oblique side 28 located at the foremost front. During the pricking operation into the predetermined portion, the front end 26 punctures the predetermined portion first, and then the portion that follows the front end 26 (namely, the portion located at the right of the front end 26 in the shown embodiment) enters the predetermined portion.

As the needle enters as described above, a circumference length of the cross section of the pricking needle that is entering (namely the total length of the peripheries that define the cross section of the needle at the pricking position) gradually increases, so as to open out the tissue below the predetermined portion, so that the wound opening is formed. In this process, it has been found, that resistance of the tissue to the entry of the pricking needle gradually increases, thus requiring the pricking needle to have a greater driving force, namely a greater pricking force since it has to enter against the resistance, thus resulting in a significant pain felt during the pricking operation.

The inventor has conceived that the resistance increase of the tissue to the entry of the pricking needle would be suppressed if an increasing extent of the circumference of the cross section of the pricking needle that enters is suppressed even with the progress of the needle entry, thus making it possible to alleviate the pain felt upon the entry of the pricking needle. And it has been found that the increase of the circumference of the cross section of the pricking needle can be suppressed by forming the rear portion of the ellipse of bent two flat surfaces as described above.

Accordingly, in the first aspect, the present invention provides a pricking needle having a front end portion as a pricking region which needle is formed from a cylindrical member (or solid cylindrical member), wherein the front end portion is defined by:
(1) a rear portion of an ellipse which is formed by cutting through the cylindrical member obliquely ahead from its backward;
(2) an isosceles triangle portion that has a central portion of a front edge of the rear portion of the ellipse as a base, that extends forward and obliquely downward from the central portion, and that extends more obliquely downward than a spreading direction of the rear portion of the ellipse;
(3) a rightward inclined surface portion that includes a right oblique side of the isosceles triangle portion, a right side portion of the front edge of the rear portion of the ellipse, and a line segment which connects a front apex of the isosceles triangle portion and a distal end of the pricking needle, and that extends from the right oblique side of the isosceles triangle obliquely downward toward the right;
(4) a leftward inclined surface portion that includes a left oblique side of the isosceles triangle portion, a left side portion of the front edge of the rear portion of the ellipse, and the line segment connecting the front apex of the isosceles triangle portion and the distal end of the pricking needle, and that extends from the left oblique side of the isosceles triangle obliquely downward toward the left; and
(5) a portion of a side face of the cylindrical member which portion includes:
   (5-1) a peripheral portion of the rear portion of the ellipse excluding the front edge thereof;
   (5-2) a peripheral portion of the rightward inclined surface portion excluding the right oblique side of the isosceles triangle, the right side portion of the front edge of the rear portion of the ellipse, and the line segment connecting the front apex of the isosceles triangle portion and the distal end of the pricking needle; and
   (5-3) a peripheral portion of the leftward inclined surface portion excluding the left oblique side of the isosceles triangle, the left side portion of the front edge of the elliptical rear portion, and the line segment connecting the front apex of the isosceles triangle and the distal end of the pricking needle.

In the second aspect, the present invention provides a pricking needle formed from a cylindrical member (or solid cylindrical member) having a front end portion as a pricking region, wherein the front end portion is defined by:
(1) an elliptical rear portion formed by cutting through a cylindrical member obliquely ahead from the backward;
(2) an line symmetrical pentagon that has a front edge of the rear portion of the ellipse as a base, that extends forward from the front edge obliquely downward, and that extends more obliquely downward than a spreading direction of the rear portion of the ellipse;
(3) a rightward inclined surface portion that includes a front right oblique side of the line symmetrical pentagon, and a line segment connecting a front apex of the line symmetrical pentagon and a distal end of the pricking region, and that extends obliquely downward toward the right from the front right oblique side of the line symmetrical pentagon;
(4) a leftward inclined surface portion that includes a front left oblique side of the line symmetrical pentagon, and a line segment connecting the front apex of the line symmetrical pentagon and the distal end of the pricking region, and that extends obliquely downward toward the left from the front left oblique side of the line symmetrical pentagon; and
(5) a portion of a side face of the cylindrical member which portion includes:
   (5-1) a peripheral portion of the rear portion of the ellipse excluding the front edge;
   (5-2) a rear left oblique side and a rear left oblique side of the line symmetrical pentagon;
   (5-3) a peripheral portion of the rightward inclined surface portion excluding the front right oblique side of the line symmetrical pentagon and the line segment that connects the front apex of the line symmetrical pentagon and the distal end of the pricking region; and
   (5-4) a peripheral portion of the leftward inclined surface portion excluding the front left oblique side of the line symmetrical pentagon and the line segment that connects the front apex of the line symmetrical pentagon and the distal end portion of the pricking region.

The present invention further provides a lancet comprising the pricking needle of the present invention that is described above and will be described below. It is noted that the lancet of the present invention may be the same as the lancet known in the part art except the pricking needle. Accordingly, more detailed descriptions of the lancet of the present invention may be omitted.

### EFFECT OF THE INVENTION

According to the present invention, the pricking needle is provided which the front end portion causing a less resistance during the pricking operation. As a result, upon obtaining a blood sample by using the lancet in which the pricking needle of the present invention, it is possible to suppress the pain felt during the pricking operation compared to the case wherein a blood sample is obtained by using a lancet in which the conventional pricking needle is used. Also, a user of the lancet that employs the pricking needle of the present invention is assured of a less psychological tension as to the pain upon pricking by recognizing beforehand that the resistance upon the pricking is lower (that is, by having such a preoccupation). As a result, the pain felt by the user during the pricking operation of the lancet can be reduced.

### BRIEF DESCRIPTION OF THE DRAWINGS

Fig. 1 is a schematic perspective view of the front end portion of the pricking needle of the prior art.
Fig. 2 is a plan view (upper drawing) and a side view (lower drawing) of the pricking needle shown in Fig. 1.
Fig. 3 is a schematic perspective view of a front end portion of a pricking needle of the first aspect of the present invention.
Fig. 4 is a plan view (upper drawing) and a side view (lower drawing) of the pricking needle shown in Fig. 3.
Fig. 5 is a schematic perspective view of a front end portion of the pricking needle of the second aspect of the present invention.
Fig. 6 is a schematic perspective view which explains a process for manufacturing the pricking needle of the present invention.

### DESCRIPTION OF REFERENCE NUMERALS

- 10:: pricking needle
- 14:: cylindrical member
- 18:: front edge of rear portion of ellipse
- 20:: apex
- 22:: right oblique side
- 26:: front end portion
- 30:: rightward inclined surface portion
- 32:: leftward inclined surface portion
- 34:: portion of side face of cylindrical member
- 36:: portion of ellipse periphery of rear portion of ellipse
- 38:: outermost periphery of rightward inclined surface portion
- 40:: outermost periphery of leftward inclined surface portion
- 50:: base
- 102:: cylindrical member
- 120:: front apex
- 150:: base portion of pricking needle
- 200:: pricking needle
- 212:: front end portion
- 226:: distal end
- a1:: right oblique side of front edge
- 12:: front end portion
- 16:: rear portion of ellipse
- 24:: left oblique side
- 28:: oblique side
- 100:: pricking needle
- 112:: front end
- 126:: distal end
- 202:: cylindrical member
- 220:: front apex
- a0:: base of front edge
- a2:: left oblique side of front edge
- a3:: portion of elliptical periphery of rear portion of ellipse
- b1:: right oblique side of isosceles triangle portion
- b2:: left oblique side of isosceles triangle portion
- c:: oblique side
- d1:: outermost periphery of rightward inclined surface portion
- d2:: outermost periphery of leftward inclined surface portion
- A0:: base of line symmetrical pentagon
- A1:: rear right oblique side of line symmetrical pentagon
- A2:: Rear left oblique side of line symmetrical pentagon
- A3:: portion of elliptical periphery of rear portion of ellipse
- B1:: front right oblique side of line symmetrical pentagon
- B2:: front left oblique side of line symmetrical pentagon
- C:: oblique side
- D1:: outermost periphery of rightward inclined surface portion
- D2:: outermost periphery of leftward inclined surface portion

### BEST EMBODIMENT FOR CARYING OUT THE INVENTION

The present invention will be described below. In the present specification, the terms "front." and "rear" are defined on the basis of the direction in which a lancet that employs the pricking needle of the present invention is launched toward a predetermined portion, namely the direction in which the pricking needle moves toward the predetermined portion. Accordingly, the term "front", "ahead" or "forward" will be used to mean the direction of the movement of the pricking needle, and "rear", "behind" or "backward" will be used to mean the opposite direction. The directions of "front" and "rear" correspond to the direction of the axis of the pricking needle, namely the direction along which the pricking needle extends, and based on this direction, the direction toward the apex 20 is referred to as "upward" and the opposite direction is referred to as "downward". That is, when the pricking needle is placed such that the base 50 lies on a flat surface as shown in Fig. 1, the term "upward" is used in order to mean the direction from the base 50 toward the apex 20, and the term "downward" is used to mean the opposite direction. Also, the terms "right" and "left" are used with reference to the direction of the pricking needle moves from the "rear" to the "front" and also in consideration of the "upward" and "downward" described above. These directions are illustrated schematically in Fig. 1 and Fig. 2, and such directions may be referenced.

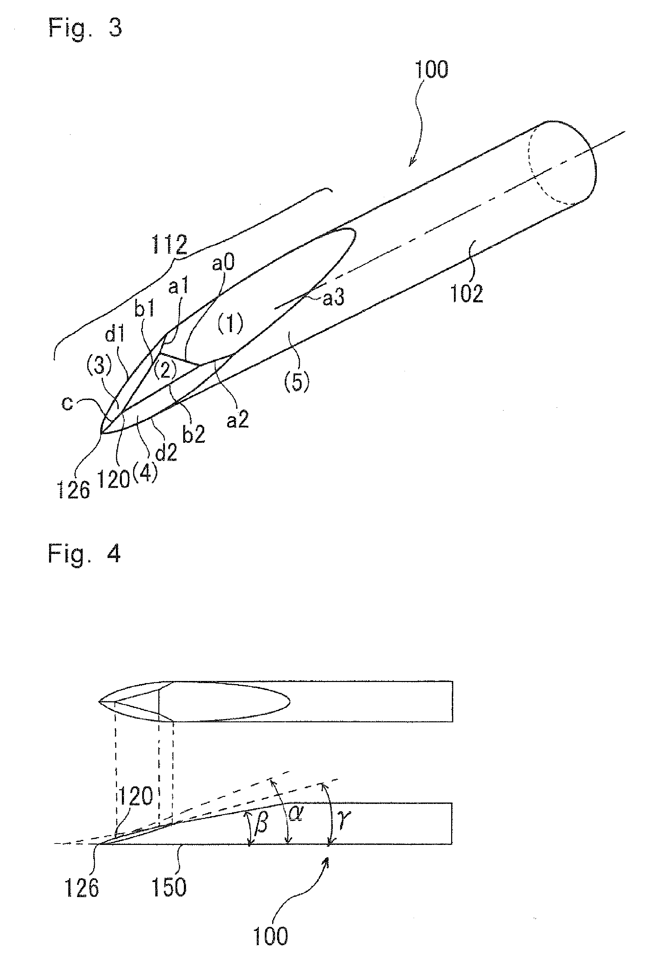

The pricking needle 100 of the first aspect according to the present invention is schematically shown in a perspective view in Fig. 3. As will be easily understood from Fig. 3, the front end portion 112 of the pricking needle of the present invention is composed of four flat surfaces, namely a flat surface (1), a flat surface (2), a flat surface (3) and a flat surface (4), and one curved surface (5).

The flat surface (1) is a rear portion of a portion of a cross section in the shape of an ellipse that appears when the cylindrical member 102 is cut obliquely ahead and downward from its rear. Such obliquely cutting may be carried out by means of a plane that inclines ahead from its rear at a predetermined angle which is not parallel to one symmetry plane of the cylindrical member (any one plane that includes the center axis of the cylindrical member), which produces an inclined cross section (which includes the flat surface (1)), and the produced cross section has an elliptical shape. It is noted that the line formed by crossing of the cross section with the symmetry plane is perpendicular to the center axis at right angles.

In the shown embodiment, the front edge of the flat surface (1) in the form of a isosceles trapezoid like shape including a top side a0 and oblique sides a1 and a2 connecting to the respective ends of the top side (which sides correspond to legs of the trapezoid), while the remaining side that defines the flat surface (1) is a portion a3 of the periphery of the ellipse. Thus, the flat surface (1) is a surface that is a rear portion of the ellipse produced by cutting the cylindrical member obliquely and ahead from its backward, and that is enclosed by the top side a0, the oblique side a1, the oblique side a2 and the portion a3 of the ellipse periphery. In other words, these sides (a0, a1 and a3) constitute the outer edge (namely, the outer periphery) of the flat surface (1), and the extension (that is, the spreading region) of the flat surface (1) is defined by these sides. The flat surface (1) extends obliquely downward relative to the axial direction (indicated by the alternate long and short dash line) of the cylindrical member from the rear toward the front.

The flat surface (2) is an isosceles triangular portion (including an equilateral triangle portion) having, as a base, the top side a0 of the central portion of the front edge of the rear portion of the ellipse (the flat surface (1)) described above. The isosceles triangular portion extends more obliquely downward than the extending direction of the flat surface (1). Therefore, as will be understood from Fig. 4 which will be referred to below, the inclination (which corresponds to the angle γ) of the flat surface (2) relative to the horizontal direction is larger than the inclination (which corresponds to the angle β) of flat surface (1).

The flat surface (3) is a rightward inclined surface portion that extends from the right oblique side b1 of the isosceles triangular portion (the flat surface (2)) described above obliquely downward toward the right, of which outer extension is defined by the right oblique side b1 of the isosceles triangular portion, the right oblique side a1 of the front edge of the rear portion of the ellipse, an oblique side c that connects the front apex 120 of the isosceles triangular portion and the distal end 126 of the pricking needle, and a line d1 which is formed by intersection of the rightward inclined surface portion that extends as described above and the side face of the cylindrical member (namely, the periphery located at the outermost position of the rightward inclined surface portion in Fig. 3).

The flat surface (4) is a leftward inclined surface portion that extends from the left oblique side b1 of the isosceles triangular portion (the flat surface (2)) described above obliquely downward toward the left, of which outer extension is defined by the left oblique side b2 of the isosceles triangular portion, the left oblique side a2 of the front edge of the rear portion of the ellipse, the oblique side c that connects the front apex 120 of the isosceles triangular portion and the distal end portion 126 of the pricking needle, and a line d2 which is formed by intersection of the leftward inclined surface portion that extends as described above and the side face of cylindrical member (namely, the periphery located at the outermost position of the leftward inclined surface portion in Fig. 3).

The curved surface (5) is a portion of the side face of the cylindrical member, which remains when the flat surfaces (1) through (4) are formed from the cylindrical member which has been cut obliquely, and includes, as the peripheries thereof:
a peripheral portion a3 of the ellipse excluding the front edges (a0, a1 and a2) of the rear portion of the ellipse;
the line d1 produced by the intersection of the rightward inclined surface portion and the side face of the cylindrical member; and
the line d2 produced by the intersection of the leftward inclined surface portion and the side face of cylindrical member.

The side d1 corresponds to a side obtained by subtracting the right oblique side b1 of the isosceles triangular portion, the right oblique side a1 of the front edge of the rear portion of the ellipse, and the oblique side c that connects the front apex 120 of the isosceles triangular portion and the distal end 126 of the pricking needle from the entire periphery (namely, the sum of the sides or the outer edge) which defines the rightward inclined surface portion (that is, d1 = sum of sides - b1-a1 - c). The side d2 corresponds to a side obtained by subtracting the left oblique side b2 of the isosceles triangular portion, the left oblique side a2 of the front edge of the rear portion of the ellipse, and the oblique side c that connects the front apex 120 of the isosceles triangular portion and the distal end portion 126 of the pricking needle from the entire periphery that defines the leftward inclined surface portion (that is, d2 = sum of sides - b2- a2 - c).

As will be easily understood, in the pricking needle of the present invention, the flat surface (1) and the flat surface (2) adjoin each other in such a configuration that bend along the top side a0 of the trapezoid, namely the base of the isosceles triangular portion. In other words, the elliptical rear portion bends so as to define the two flat surfaces. The pricking needle 100 of the present invention having the constitution described above is shown in a side view of Fig. 4, similarly to Fig. 2. In the shown embodiment, the pricking angle α of the pricking needle is the same as that in the case shown in Fig. 2, and the inclination angle β of the flat surface (1) (namely, in the side view of the pricking needle as shown in Fig.3, the angle formed by the flat surface (1) and the base portion 150 of the pricking needle (i.e., a profile line segment in the longitudinal direction of the pricking needle shown at the bottom in the side view of the cylindrical member when viewed sideways such that the distal end of the pricking needle appears at the left side), that is, the inclination angle of the flat surface (1) relative to the axis of the cylindrical member) is equal to the inclination angle β of the rear portion 16 of the ellipse. In Fig. 3, the pricking angle of α is available in a region between the distal end 126 and the front apex 120 of the isosceles triangular portion, and the distance therebetween is clearly shorter than the distance between the dital end 26 and the apex 20 shown in Fig. 2.

In the pricking needle of the present invention, in a region located behind the front apex 120 and ahead of the flat surface (1), namely in the flat surface (2), the inclination angle is γ and satisfies a relationship of β < γ < α. During the process in which the pricking needle penetrates the body tissue, the pricking needle expands the tissue at the inclination angle, and therefore the inclination angle has a similar meaning to the pricking angle. The conventional pricking needle enters the human body up to a point just before the rear portion 16 while keeping the pricking angle α, before entering at the inclination angle β. In contrast, the pricking needle of the present invention enters at the pricking angle α over a shorter distance, followed by the entry of the flat surface (2) that has the inclination angle γ which is smaller than α and larger than β, and upon arrival at the flat surface (1), the pricking needle enters at the inclination angle β.

As a result, during the process of entering at the inclination angle γ, since the pricking angle is smaller than the inclination angle (α) of the conventional pricking needle, the pricking needle of the present invention causes less pain in the pricking operation. In other words, in case the inclination angle of the flat surface is γ, the increase in the periphery of the cross section of the pricking needle is less than in the case where the inclination angle is α. As a result, the resistance of the body tissue to the penetration of the pricking needle does not increase so much as in the case where the pricking needle shown in Fig. 2 is used, so that the pain felt upon the entry of the pricking needle is alleviated when compared with the case where the pricking needle shown in Fig. 2 is used.

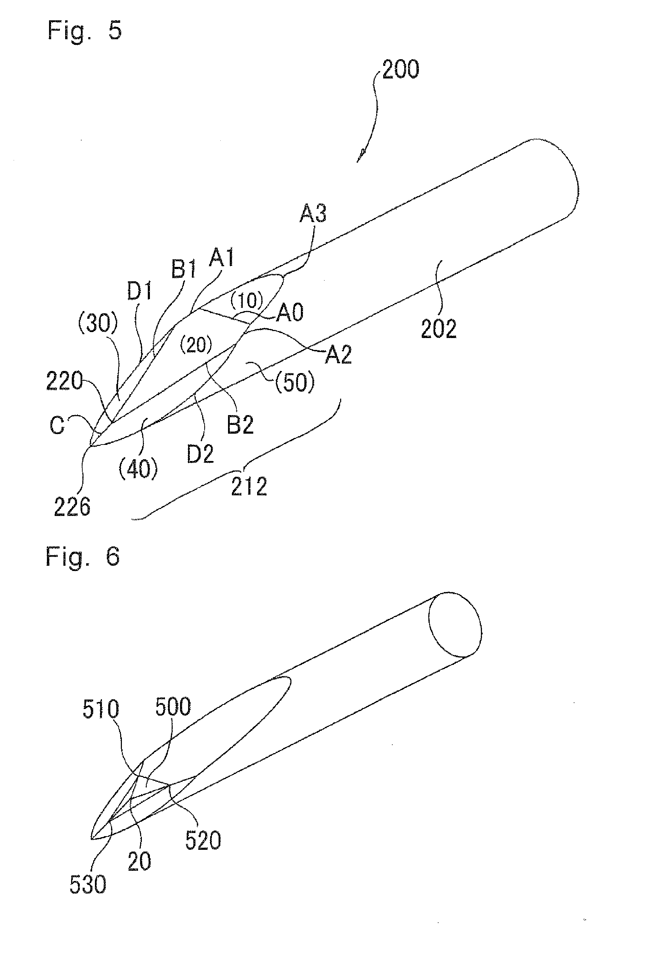

A pricking needle 200 according to the second aspect of the present invention is shown schematically in a perspective view of Fig. 5. As will be easily understood from Fig. 5, the distal end 212 of the pricking needle of the present invention is composed of four flat surfaces, namely a flat surface (10), a flat surface (20), a flat surface (30) and a flat surface (40), and one curved surface (50).

The flat surface (10) is a rear portion of an elliptical cross section that appears when the cylindrical member 202 is cut obliquely ahead from its rear. Such obliquely cutting is carried out as described above. In the illustrated embodiment, the front edge of the flat surface (10) constitutes a base A0 of the flat surface (20) that is an line symmetrical pentagonal portion which will be described later.

The flat surface (20) is the line symmetrical pentagonal portion that has, as a base, the front edge of the rear portion of the ellipse (the flat surface (10)), that extends obliquely downward and forward from said front edge, and that extends more obliquely downward than the spreading direction of the rear portion of the ellipse (the flat surface (10)) similarly to the pricking needle according to the first aspect of the present invention. That is, the line symmetrical pentagonal portion is composed of the base A0, a rear right oblique side A1 and a rear left oblique side A2, and a front right oblique side B1 and a front left oblique side B2. The term "line symmetrical" means that a line passing the foremost apex 220 of the line symmetrical pentagon which line is perpendicular to the base A0 corresponds to the axis of the line symmetry. It is noted that strictly speaking, the rear oblique sides are curved and therefore the flat surface (20)) has an approximately pentagon shape.

The flat surface (30) is a rightward inclined surface portion that extends obliquely downward toward the right from the front right oblique side B1 of the line symmetrical pentagonal portion (the flat surface (20)), of which outer extension is defined by the front right oblique side B1 of the line symmetrical pentagonal portion, the oblique side C that connects the foremost apex 220 of the line symmetrical pentagon and the distal end 226 of the pricking needle, and a line D1 which is formed by intersection of the rightward inclined surface portion that extends as described above and the side face of the cylindrical member (namely, the periphery located at the outermost position of the rightward inclined surface portion in Fig. 5).

The flat surface (40) is a leftward inclined surface portion that extends obliquely downward toward the left from the front left oblique side B2 of the line symmetrical pentagonal portion (the flat surface (20)), of which outer extension is defined by the front left oblique side B2 of the line symmetrical pentagonal portion, the oblique side C that connects the foremost apex 220 of the line symmetrical pentagon and the distal end portion 226 of the pricking needle, and a line D2 which is formed by intersection of the leftward inclined surface portion that extends as described above and the side face of cylindrical member (namely, the periphery located at the outermost position of the leftward inclined surface portion in Fig. 5).

The curved surface (50) is a portion of the side face of the cylindrical member, which remains when the flat surfaces (1) through (4) are formed from the cylindrical member which has been cut obliquely, and includes, as the peripheries thereof:
a peripheral portion A3 of the rear portion of the ellipse excluding the front edge A0 of the rear portion of the ellipse;
the rear right oblique side A1 and the rear left oblique side A2 of the line symmetrical pentagonal portion;
the line D1 produced by the intersection of the rightward inclined surface portion and the side face of the cylindrical member; and
the line D2 produced by the intersection of the leftward inclined surface portion and the side face of the cylindrical member.

The line D1 corresponds to a side obtained by subtracting the front right oblique side B1 of the line symmetrical pentagonal portion, and the oblique side C that connects the foremost apex 220 of the line symmetrical pentagonal portion and the distal end 226 of the pricking needle from the entire periphery that defines the rightward inclined surface portion. Also, the line D2 corresponds to a side obtained by subtracting the front left oblique side B2 of the line symmetrical pentagonal portion and the oblique side C that connects the foremost apex 220 of the line symmetrical pentagonal portion and the distal end 226 of the pricking needle from the entire periphery that defines the leftward inclined surface portion.

As will be easily understood, in the pricking needle of the present invention, the flat surface (10) and the flat surface (20) adjoin each other in such a configuration that bends along the base A0, namely the most proximate side of the line symmetrical pentagon. Also in this case, the pricking angle is the same as the inclination angle of the flat surface (10) similarly to the case of the pricking needle of the present invention shown in Fig. 3. That is, the pricking angle is α and the inclination angle of the flat surface (10) is β. In the pricking needle shown in Fig. 5, the inclination angle of the flat surface (20) is γ' (not shown), which also has an angle between α and β. However, a relationship of γ' < γ is applicable since the flat surface (20) extends longer along the longitudinal direction of the pricking needle. The above mentioned explanation made with reference to Fig. 3 applies also to this case, in that the pricking needle shown in Fig. 5 is capable of mitigating the pain felt during the pricking operation compared to the case of the conventional pricking needle.

The pricking needle of the present invention can be manufactured by, for example, such a process as follows. First, the conventional pricking needle such as the pricking needle 10 shown in Fig. 1 is manufactured, by any appropriate method. The pricking needle of the present invention as shown in Fig. 3 may be obtained by removing a portion of triangular pyramid portion 500 having an apex at the point 20 from the pricking needle 10 thus obtained. Such state is schematically shown in Fig. 6, which shows substantially the same as Fig. 1, except for showing the triangular pyramid portion 500 to be removed. The triangular pyramid portion 500 has one apex at the point 20 and three other apexes (510, 520 and 530) located on certain points on the side 22, the side 24 and the side 28, respectively. It is noted that it is preferable to make the distance between the apex 510 and the apex 20 equal to the distance between the apex 520 and the apex 20. The inclination angle of the flat surface (2) can be changed by variously selecting the apex 510, the apex 520 and the apex 530. Such removal can be done, for example, by grinding off the portion of the apex 20 of the conventional pricking needle.

The apex 510 and the apex 520 may be located at positions more backward compared with the state shown in Fig. 6, and on the peripheral portion 36 of the rear portion of the ellipse. A modified triangular pyramid portion (or a quasi triangular pyramid portion) having apex at the point 20 over a base plane defined by the apex 510, the apex 520 and the apex 530 is removed, thereby to obtain the pricking needle of the present invention shown in Fig. 5.

Thus, the pricking needle of the present invention can be manufactured by the process comprising the steps of:
(1) cutting a cylindrical material obliquely ahead so as to form an elliptical cross section;
(2) cutting a front portion of the elliptical cross section thus formed in a direction to the right obliquely downward and in a direction to the left obliquely downward so as to form a rightward inclined surface and a leftward inclined surface so as to obtain a pricking needle precursor that has the remaining portion of the elliptical cross section and the rightward inclined surface and the leftward inclined surface (it is noted that when viewing the pricking needle precursor ahead from the rear of the elliptical cross section forward, the rightward inclined surface and the leftward inclined surface are disposed in a line symmetric relationship with respect to the axis of the cylindrical material, and the rear portion of the elliptical cross section adjoins the rightward inclined surface and the leftward inclined surface so as to form the apex defined by these three members (the rightward inclined surface, the leftward inclined surface, and the rear portion of the elliptical cross section) that are in contact with each other), and
(3) removing a portion of triangular pyramid portion, that is defined by a point located on a contact line between the rightward inclined surface and the rear portion of the elliptical cross section, a point located on a contact line between the leftward inclined surface and the rear portion of the elliptical cross section, a point located on a contact line between the rightward inclined surface and the leftward inclined surface and said apex, from the pricking needle precursor.

As will be easily understood, the pricking needle as shown in Fig. 1 is obtained by the steps (1) and (2), and the portion of triangular pyramid portion is removed from such pricking needle in the step (3). Therefore, in the step (1), the elliptical cross section of which portion corresponds to the surface (a) is formed, and in the step (2), the rightward inclined surface (30 or b) and the leftward inclined surface (32 or c) are formed, so as to obtain the apex 20. Thus the pricking needle shown in Fig. 1 is obtained. Then, the triangular pyramid portion (that corresponds to 500 in Fig. 6) is removed so that the pricking needle of the present invention is obtained.
Thus, in the step (2), the remaining portion of the elliptical cross section corresponds to the flat surface (a), and the pricking needle precursor corresponds to the pricking needle shown in Fig. 1. As to the pricking needle precursor, the rightward inclined surface (b) and the leftward inclined surface (c) are disposed line symmetrically with respect to the oblique side 28 formed by contact between these surfaces when viewed forward from the rear portion of the elliptical cross section. Lines formed by the contact of the rear portion of the elliptical cross section with the rightward inclined surface and the leftward inclined surface are the right oblique side 22 and the left oblique side 24, respectively.

In the step (3), the point located on the contact line between the rightward inclined surface and the rear portion of the elliptical cross section may be located on the right side peripheral portion that defines the rear portion of the elliptical cross section, and the point located on the contact line between the leftward inclined surface and the rear portion of the elliptical cross section may be located on the right side peripheral portion that defines the rear portion of the elliptical section, in which case the portion of the quasi triangular pyramid portion is removed, thereby the pricking needle of the present invention shown in Fig. 5 being manufactured.

The pricking needle of the present invention can be manufactured by using a cylindrical material commonly used for the pricking needle of the lancet, such as a metal wire, preferably a stainless steel wire.

It is noted that the pricking needle of the present invention has been described in which the flat surfaces and the curved surface that define the front end portion of the pricking needle define clear border lines. However, the border lines may not necessarily be clear, and the they may be rounded into a chamfered surface without a corner. As the corners are eliminated, it is expected that the pain would be further mitigated.

In the pricking needle of the present invention, there is no particular restriction on the pricking angle α, which may be similar to that of the pricking needle commonly used. The pricking angle α may be, for example, from 5° to 30°, preferably from 5° to 20°, more preferably from 8° to 15° and particularly from 9° to 12°.

There is also no particular restriction on the inclination angle β of the flat surface (1) or the flat surface (10), which may be similar to that of the pricking needle commonly used. The inclination angle α may be, for example, from 5° to 20°, preferably from 5° to 13°, more preferably from 5° to 10° and particularly from 6° to 8°.

There is also no particular restriction on the inclination angles γ or γ' of the flat surface (2) and the flat surface (20), respectively, which may be, for example, from 5° to 20°, preferably from 5° to 15°, more preferably from 7° to 14° and particularly from 9° to 12°. It is needless to say that the angles α, β and γ (or γ') must satisfy the relationships of inequality described above.

There are also no particular restriction on the material and the diameter of the pricking needle, which may be those commonly used for the pricking needle of the lancet. Specifically, the diameter of the cylindrical member made of the stainless steel is, for example, from 2.0 to 0.2 mm, preferably from 1.5 to 0.4 mm and more preferably from 1.0 to 0.4 mm.

### INDUSTRIAL APPLICABILITY

The pricking needle of the present invention described above can be used when manufacturing a lancet, and the lancet thus manufactured is capable of mitigating the pain felt during pricking operation.

## Claims

1. A pricking needle having a front end portion as a pricking region which needle is formed from a cylindrical member, wherein the front end portion is defined by:
(1) a rear portion of an ellipse which is formed by cutting through the cylindrical member obliquely ahead from its backward;
(2) an isosceles triangle portion that has a central portion of a front edge of the rear portion of the ellipse as a base, that extends forward and obliquely downward from the central portion, and that extends more obliquely downward than a spreading direction of the rear portion of the ellipse;
(3) a rightward inclined surface portion that includes a right oblique side of the isosceles triangle portion, a right side portion of the front edge of the rear portion of the ellipse, and a line segment which connects a front apex of the isosceles triangle portion and a distal end of the pricking needle, and that extends from the right oblique side of the isosceles triangle obliquely downward toward the right;
(4) a leftward inclined surface portion that includes a left oblique side of the isosceles triangle portion, a left side portion of the front edge of the rear portion of the ellipse, and the line segment connecting the front apex of the isosceles triangle portion and the distal end of the pricking needle, and that extends from the left oblique side of the isosceles triangle obliquely downward toward the left; and
(5) a portion of a side face of the cylindrical member which portion includes:
(5-1) a peripheral portion of the rear portion of the ellipse excluding the front edge thereof;
(5-2) a peripheral portion of the rightward inclined surface portion excluding the right oblique side of the isosceles triangle, the right side portion of the front edge of the rear portion of the ellipse, and the line segment connecting the front apex of the isosceles triangle portion and the distal end of the pricking needle; and
(5-3) a peripheral portion of the leftward inclined surface portion excluding the left oblique side of the isosceles triangle, the left side portion of the front edge of the elliptical rear portion, and the line segment connecting the front apex of the isosceles triangle and the distal end of the pricking needle.

2. The pricking needle according to claim 1 wherein a pricking angle is in the range between 5° and 30°.

3. The pricking needle according to claim 1 or 2 wherein an inclination angle of the rear portion of the ellipse is in the range between 5° and 20°.

4. The pricking needle according to any one of claims 1 to 3 wherein an inclination angle of the isosceles triangle portion is in the range between 5° and 25°.

5. A lancet which comprises the pricking needle according to any one of claims 1 to 4.

6. A pricking needle formed from a cylindrical member having a front end portion as a pricking region, wherein the front end portion is defined by:
(1) an elliptical rear portion formed by cutting through a cylindrical member obliquely ahead from the backward;
(2) an line symmetrical pentagon that has a front edge of the rear portion of the ellipse as a base, that extends forward from the front edge obliquely downward, and that extends more obliquely downward than a spreading direction of the rear portion of the ellipse;
(3) a rightward inclined surface portion that includes a front right oblique side of the line symmetrical pentagon, and a line segment connecting a front apex of the line symmetrical pentagon and a distal end of the pricking region, and that extends obliquely downward toward the right from the front right oblique side of the line symmetrical pentagon;
(4) a leftward inclined surface portion that includes a front left oblique side of the line symmetrical pentagon, and a line segment connecting the front apex of the line symmetrical pentagon and the distal end of the pricking region, and that extends obliquely downward toward the left from the front left oblique side of the line symmetrical pentagon; and
(5) a portion of a side face of the cylindrical member which portion includes:
(5-1) a peripheral portion of the rear portion of the ellipse excluding the front edge;
(5-2) a rear left oblique side and a rear left oblique side of the line symmetrical pentagon;
(5-3) a peripheral portion of the rightward inclined surface portion excluding the front right oblique side of the line symmetrical pentagon and the line segment that connects the front apex of the line symmetrical pentagon and the distal end of the pricking region; and
(5-4) a peripheral portion of the leftward inclined surface portion excluding the front left oblique side of the line symmetrical pentagon and the line segment that connects the front apex of the line symmetrical pentagon and the distal end portion of the pricking region.

7. The pricking needle according to claim 6 wherein a pricking angle is in the range between 5° and 30°.

8. The pricking needle according to claim 6 or 7 wherein an inclination angle of the rear portion of the ellipse is in the range between 5° and 20°.

9. The pricking needle according to any one of claims 6 to 8 wherein an inclination angle of the line symmetry pentagon is in the range between 5° and 25°.

10. A lancet which comprises the pricking needle according to any one of claims 6 to 9.

11. A process for manufacturing a pricking needle comprising the steps of:
(1) cutting a cylindrical material obliquely ahead so as to form an elliptical cross section;
(2) cutting a front portion of the elliptical cross section thus formed in a direction to the right obliquely downward and in a direction to the left obliquely downward so as to form a rightward inclined surface and a leftward inclined surface so as to obtain a pricking needle precursor that has the remaining portion of the elliptical cross section and the rightward inclined surface and the leftward inclined surface (it is noted that when viewing the pricking needle precursor ahead from the rear of the elliptical cross section forward, the rightward inclined surface and the leftward inclined surface are disposed in a line symmetric relationship with respect to the axis of the cylindrical material, and the rear portion of the elliptical cross section adjoins the rightward inclined surface and the leftward inclined surface so as to form the apex defined by these three members (the rightward inclined surface, the leftward inclined surface, and the rear portion of the elliptical cross section) that are in contact with each other), and
(3) removing a portion of triangular pyramid portion, that is defined by a point located on a contact line between the rightward inclined surface and the rear portion of the elliptical cross section, a point located on a contact line between the leftward inclined surface and the rear portion of the elliptical cross section, a point located on a contact line between the rightward inclined surface and the leftward inclined surface and said apex, from the pricking needle precursor.

12. A lancet which is produced by the process according to claim 11.
